# EUROPEAN PATENT APPLICATION

(11) **EP 2 653 450 A1**
(43) Date of publication of application: **23.10.2013**
(21) Application number: 11848966.5
(22) Date of filing: 15.12.2011
(51) Int. Cl.: C02F 3/34, C12N 1/20, C12N 11/14

(54) **BIOPREPARATION FOR PURIFYING WATER FROM HYDROCARBON CONTAMINANTS**

(30) Priority: 17.12.2010 RU 2010151939
(71) Applicant: Khlynovskiy, Alexey Mikhailovich, St.Petersburg 197198 (RU)
(72) Inventor: GORDIENKO, Irina Vladimirovna, St.Petersburg 191028 (RU); ANDREEVA, Nadezhda Vladimirovna, St.Petersburg 195112 (RU); BOIKOVA, Irina Vasilievna, St.Petersburg 192283 (RU); NOVIKOVA, Irina Igorevna, St.Petersburg 197343 (RU); KOZLOVA, Marina Yurievna, St.Petersburg 194356 (RU)
(74) Representative: Gulde Hengelhaupt Ziebig & Schneider
(86) International application number: PCT/RU2011/000987
(87) International publication number: WO 2012/082016

(57) **Abstract**

The invention relates to agents for removing organic contaminants from environmental media and is intended, in particular, for the biological purification of water contaminated with petroleum and petroleum products. The biopreparation for purifying water from hydrocarbon contaminants, comprising a porous carrier of biologically active microorganisms and containing the glass-like metaphosphates KPO₃, Mg(PO₃)₂, Ca(PO₃)₂, as well as Al₂O₃ and hydrocarbon-decomposing microorganisms, additionally contains TiO₂ and inorganic nitrogen-containing compounds in the following component ratio (wt%): KPO₃ - 20-40; Mg(PO₃)₂ - 5-10; Ca(PO₃)₂ - 30-50; Al₂O₃ - 5-10; TiO₂ - 1-5; KNO₃ - 1-3; NH₄H₂PO₄ - the remainder. The result is an increase in the effectiveness of the biopreparation in purifying water from hydrocarbon contaminants and an increase in the active life of the biopreparation.

## Description

### Technical field

The invention relates to agents for removing organic contaminants from environmental media and is intended, in particular, for the biological purification of water contaminated with petroleum and petroleum products.

### Background art

A known agent for biological purification of water contaminated with phenols and phenolic compounds comprises a porous ceramic carrier of biologically active microorganisms with branched open porous structure having surface and deep pores therein with total porosity 77-91 %, wherein said carrier contains a monoculture of microorganisms artificially immobilized in the ceramics pores that has been chosen to match the type of contamination, with the following component ratio (wt%):
monoculture of microorganisms - 0.01-10.0
porous ceramics - the remainder,
see RU 2107665 C1.

The disadvantage of this technical solution consists in the passivity of the carrier of bacteria that does not provide nutrients for the bacteria, which results in low oxidative activity of the organic substance-decomposing microorganisms.

A known biopreparation for purifying water from hydrocarbon contaminants comprises a porous carrier of biologically active microorganisms and contains the glass-like metaphosphates KPO₃, Mg(PO₃)₂, Ca(PO₃)₂, as well as Al₂O₃ and hydrocarbon-decomposing microorganisms chosen to match the type of contamination, see RU 2181701 2. The preparation also contains the following microelements: Zn, Mn, Fe, Cu, Co, Ni, Mo, V, Se, Ag.

This technical solution has been taken as a prototype of the present invention.

The disadvantage of the prototype consists in the fact that Cu, Se, Fe manifest toxic properties towards microorganisms, all the more so in the concentrations mentioned in the description of RU 2181701 C2, and Ag has a bactericidal effect. The abovementioned microelements can be used as plant fertilizer but they are useless for the microorganisms, and Cu, Se, Fe, Ag are harmful for the microorganisms, which significantly reduces the effectiveness of the known biopreparation; nutrients for the microorganisms are provided only by metaphosphates, which is insufficient, arrests the development of microorganisms and negatively affects their biological activity.

In addition the prototype biopreparation dissolves in the water medium within not more than 30 days, after which the effect of the preparation ceases. Relatively fast dissolution of the preparation is caused by the fact that it contains Si03 and B₂O₃, which affect the dissolution kinetics by increasing its rate. Al₂O₃, when included in the low concentration specified in the prototype (0.1-5%) does not ensure the required slowing down of metaphosphates' dissolution in the water.

### Summary of the invention

It is an object of the present invention to increase the effectiveness of the biopreparation for purifying water from hydrocarbon contaminants and also to increase the active life thereof.

According to the invention there is provided a biopreparation for purifying water from hydrocarbon contaminants, comprising a porous carrier of biologically active microorganisms and containing the glass-like metaphosphates KPO₃, Mg(PO₃)₂, Ca(PO₃)₂, as well as Al₂O₃ and hydrocarbon-decomposing microorganisms, which additionally contains TiO₂ and inorganic nitrogen-containing compounds in the following component ratio (wt%):

| | |
|---|---|
| KPO₃ | - 20-40 |
| Mg(PO₃)₂ | - 5-10 |
| Ca(PO₃)₂ | - 30-50 |
| Al₂O₃ | - 5-10 |
| TiO₂ | - 1-5 |
| KNO₃ | - 1-3 |
| NH₄H₂PO₄ | - the remainder. |

The applicant has not found any sources of information containing data on technical solutions identical to the present invention, which enables to conclude that the invention conforms to the criterion "Novelty" (N).

Implementation of the features of the invention provides the biopreparation with new important properties, in particular, TiO₂ ensures the prevalence of amorphous phase in the porous carrier of biologically active microorganisms and the reduction of the amount of undesirable crystalline phase that is practically non-soluble. This makes it possible to significantly increase the biopreparation effectiveness. The presence of NH₄H₂PO₄ ensures effective foaming of the pellets.

The applicant has not found any sources of information containing data on the influence of the features of the invention on the technical result produced by their implementation. In applicant's opinion, this enables to conclude that the present technical solution conforms to the criterion "Inventive Step" (IS).

### Brief description of the drawings

The invention is further explained, by way of detailed description of examples of its embodiments, without reference to any drawings.

### Preferred embodiment

The porous carrier of biologically active microorganisms is manufactured as follows. Molten glass-like metaphosphates KPO₃, Mg(PO₃)₂, Ca(PO₃)₂, as well as Al₂O₃, TiO₂ are subjected to fme grinding in a disk grinder like ID-175 manufactured by NPO Mekhanobr-Tekhnika (Russia, St. Petersburg). Then the obtained powdered components are mixed and KNO₃ is added thereto, which is the main source of nitrogen for microorganisms' nutrition, also ammonium monophosphate (NH₄H₂PO₄) is added in ground form with the same granulometric parameters.

Ammonium monophosphate acts as a very effective foaming agent and also serves as a secondary source of nitrogen. The obtained mixture is granulated in a palletizing machine like OT manufactured by OOO Feniks (Russia). The obtained pellets are dried in a desiccator at a temperature of 80-100 °C for at least two hours. Then the pellets are placed into a tunnel furnace, in this particular example manufactured by Teplizol (Russia, St. Petersburg), and heated to a temperature of 520-550 °C with heating rate 15-20 °C per minute. At this time the pellets are foamed and the pores are formed. The density of obtained pellets equals 0.3-0.6 g/cm³, porosity equals 2500-3200 cm²/g.

When the content of KPO₃ is less than 20 wt% and the content of Mg(PO₃)₂ is less than 5 wt%, the microorganisms do not receive sufficient nutrients. When the contents of these components are greater than 40 and 10 wt%, respectively, the structure of the carrier is not uniform enough.

When the content of Ca(PO₃)₂ is less than 30 wt%, the required solubility of pellets is not ensured. When the content of Ca(PO₃)₂ is greater than 50 wt%, the carrier has undesirable crystalline structure.

When the content of Al₂O₃ is less than 5 wt%, the rate of metaphosphates' dissolution in water is too high and therefore the effect of the biopreparation ceases in a relatively short time due to dissolution of the carrier.

The content of All₂O₃ above 10 wt% is impractical, since it practically stops having an effect on the rate of dissolution of glass-like metaphosphates in water.

The presence of TiO₂ ensures the prevalence of amorphous (glass-like) phase in the product. When this component is absent, there exists a significant amount of undesirable crystalline phase that is practically insoluble, which considerably reduces the biopreparation effectiveness. When the content of TiO₂ is greater than 10 wt%, the specified effect practically stays the same (is not increased).

Biologically active microorganisms are introduced into the porous carrier by placing the carrier in a culture fluid containing microorganisms with titer of (2-5)* 10⁹ cells/mL. Surface cultivation of microorganisms in the porous carrier was conducted at 20 °C for 72 hours. After cultivation the titer of the obtained biopreparation amounted to 10¹² viable cells per 1 g.

The cultivation process was performed in a fermenter in aerobic conditions during 36 hours. 0.02 % crude petroleum was used as the inducer.

Petroleum-oxidizing ability of the biopreparation was determined as follows.

### Example 1 - LABORATORY TESTS.

The tests use biopreparations comprising a porous carrier that contains the following components (wt%):

| | |
|---|---|
| KPO₃ | - 20 |
| Mg(PO₃) | - 5 |
| Ca(PO₃) | - 50 (alternative I) |
| Al₂O₃ | - 10 |
| TiO₂ | - 1 |
| KNO₃ | - 1 |
| NH₄H₂PO₄ | - the remainder (13) |
| | |
| KPO₃ | - 40 |
| Mg(PO₃) | - 10 |
| Ca(PO₃) | - 30 (alternative II) |
| Al₂O₃ | - 5 |
| TiO₂ | - 5 |
| KNO₃ | - 3 |
| NH₄H₂PO₄ | - the remainder (7) |
| KPO₃ | - 30 |
| Mg(PO₃) | - 7 |
| Ca(PO₃) | - 40 (alternative III) |
| Al₂O₃ | - 8 |
| TiO₂ | - 3 |
| KNO₃ | - 2 |
| NH₄H₂PO₄ | - the remainder (10) |

Hydrocarbon-decomposing microorganisms in all examples were embodied as bacterial strains Burkholderia caryophylli HT-4 and Psendomonas fleuorescens biovar II HT-6 isolated from soils contaminated with petroleum products, at selective culture media that have petroleum product hydrocarbons as the only source of hydrocarbon nutrients.

The strains are registered in the collection of microorganisms of the All-Russian Research Institute of Plant Protection (VIZR). The strains are stored separately in an agarized culture medium at 0÷+5 °C or in a lyophilized state in ampoules.

### Strain Burkholderia caryophylli HT-4.

### Cultural and morphological characteristics of the strain.

Motile, straight rods sized 0.8-0.9×0.9-2; 1.5×1.5 µm, occurring singly or in clusters. In standard culture media (MPA, BBL, Becton Dickinson) it forms round colonies 2 mm in diameter, convex, smooth, glistening, mucoid, the margin is entire, white, opaque. Gram staining and Gram reaction using Gregensen method (1978) - gram-negative.

### Biochemical characteristics of the strain.

Aerobic. Chemoorganotrophic. Does not need growth factors. Oxidase test - positive. Catalase test - positive. Does not produce fluorescent pigment. Does not grow at +4 °C. Grows at +41 °C. Has denitrification ability with formation of N₂. Does not hydrolyze gelatin. Does not hydrolyze starch. Uses glucose, glutamate, ethanol, succinate, xylose, mannitol as the sole carbon source. Uses hydrocarbons as a source for growth.

### Strain Pseudomonas fluorescens biovar II HT-6.

### Cultural and morphological characteristics of the strain.

Small straight single rods, slightly curved, with polar flagella, do not produce spores. Size 0.8-0.9×1.1-3.0 µm. In standard culture media (MPA, BBL, Becton Dickinson) it forms round colonies 2 mm in diameter or more, convex, smooth, glistening, the margin is entire, colourless, opaque. Gram staining and Gram reaction using Gregensen method (1978) - gram-negative.

### Biochemical characteristics of the strain.

Aerobic, able to grow in anaerobic conditions in the presence of nitrates in culture medium. Chemoorganotrophic. Type of oxidative metabolism in the test - O/F. Grows at 4 °C, does not grow at 41 °C. Catalase-positive.

2 ml of different petroleum products (crude petroleum, diesel oil, mazut and transformer oil) were introduced into plastic cuvettes 21x34 cm divided into 5 groups of 4 units each and filled with 3 1 of water. Then biopreparations in the amount of 3 g were applied to the water surface in cuvettes of groups 1-4, placing biopreparation with carrier I into the 1^{st} cuvette of each group, biopreparation with carrier II into the 2^{nd} cuvette of each group, biopreparation with carrier III into the 3^{rd} cuvette of each group and the prototype preparation into the 4^{th} cuvette of each group.

The 5^{th} group of cuvettes with water was used as a reference: the abovementioned contaminants were also added thereto but the purification agents were not added.

3-4 days later after applying the biopreparation to the water surface contaminated with petroleum products a bacterial film was formed around the biopreparation particles, which indicates that it serves as a nutrient for the microorganisms. After 1 month the iridescent film upon water surface was completely gone. After 2 months from the experiment start the concentration of petroleum products in the water was determined using the method of IR spectrophotometry in device "AN-2".

Test results are shown in Table 1.

### Example 2 - FIELD TESTS.

The tests were performed in a 25 m³ well of a sewage treatment facility USV-M-20 (TU 4859-001-1121534-2002) installed at the territory of the state unitary enterprise SPb GUP Mostotrest (Russia, St. Petersburg). Prior to introduction of the biopreparation a sample of sewage waters was taken from the well in order to determine the concentration of hydrocarbons. The results of analysis showed that the initial content of a mixture of different petroleum products in the well amounted to 0.63 mg/dm³. After taking the water sample the inventive biopreparation was introduced into the well in the amount of 0.5 kg.

After 12 and 40 days samples for analysis were taken and then 0.5 kg of the biopreparation were additionally introduced into the well.

The test results showed that during 40 days of the test the process of microbiological oxidation of petroleum products contained in the sewage waters of the production facilities of SpbGUP Mostotrest remained active, the concentration of hydrocarbons was reduced to 0.26 mg/dm³. Notwithstanding the constant inflow of petroleum products into the sewage waters during the test, the effectiveness of purification amounted to ∼60 %.

The contents of petroleum products in the water samples were analyzed in the certified testing laboratory center of a branch of federal state healthcare institution FGUZ Sanitary and Epidemiological Center in St. Petersburg.

The following measurement tools were used: fluid analyzer "Fluorat-02" and spectrophotometer AN-2 ND.

Thus, both laboratory and field tests confirm high effectiveness of the inventive biopreparation.

### Industrial applicability

The invention can be implemented by means of known materials and equipment. In applicant's opinion, this enables to conclude that the inventions conform to the criterion "Industrial Applicability" (IA).

**Table 1**

| Study Results | | | | | |
|---|---|---|---|---|---|
| Cuvette group | Cuvette No. | Type of cantamination | Initial content of oil products in the water, mg/dm³ | Final content of oil products in the water, mg/dm³ | Purificati on effectiveness, % |
| 1 (carrier I) | 1 | Crude oil | 0.17 | 0.04 | 76.4 |
| | 2 | Diesel fuel | 0.30 | 0.09 | 70.0 |
| | 3 | Mazut | 0.22 | 0.06 | 72.7 |
| | 4 | Transformer oil | 0.32 | 0.07 | 78.1 |
| | | | | | |
| 2 (carrier II) | 1 | Crude oil | 0.17 | 0.04 | 76.4 |
| | 2 | Diesel fuel | 0.30 | 0.09 | 70.0 |
| | 3 | Mazut | 0.22 | 0.06 | 72.7 |
| | 4 | Transformer oil | 0.32 | 0.07 | 78.1 |
| | | | | | |
| 3 (carrier III) | 1 | Crude oil | 0.17 | 0.03 | 82.3 |
| | 2 | Diesel fuel | 0.30 | 0.08 | 73.3 |
| | 3 | Mazut | 0.22 | 0.04 | 81.8 |
| | 4 | Transformer oil | 0.32 | 0.06 | 75.0 |
| | | | | | |
| 4 (prototype) | 1 | Crude oil | 0.17 | 0.06 | 64.7 |
| | 2 | Diesel fuel | 0.30 | 0.11 | 63.3 |
| | 3 | Mazut | 0.22 | 0.07 | 68.2 |
| | 4 | Transformer oil | 0.32 | 0.09 | 71.9 |
| | | | | | |
| 5 (reference) | 1 | Crude oil | 0.17 | 0.16 | 5.8* |
| | 2 | Diesel fuel | 0.30 | 0.28 | 6.66* |
| | 3 | Mazut | 0.22 | 0.21 | 4.5* |
| | 4 | Transformer oil | 0.32 | 0.30 | 6.25* |

| | | | | | |
|---|---|---|---|---|---|
| *- Self-purification | | | | | |

## Claims

1. Biopreparation for purifying water from hydrocarbon contaminants, which comprises a porous carrier of biologically active microorganisms and contains the glass-like metaphosphates KPO₃, Mg(PO₃)₂, Ca(PO₃)₂, as well as Al₂O₃ and hydrocarbon-decomposing microorganisms, **characterized in that** t it additionally contains TiO₂ and inorganic nitrogen-containing compounds in the following component ratio (wt%):
| | | |
|---|---|---|
| KPO₃ | - | 20-40 |
| Mg(PO₃)₂ | - | 5-10 |
| Ca(PO₃)₂ | - | 30-50 |
| Al₂O₃ | - | 5-10 |
| TiO₂ | - | 1-5 |
| KNO₃ | - | 1-3 |
| NH₄H₂PO₄ | - | the remainder. |
